# EUROPEAN PATENT SPECIFICATION

(11) **EP 1 817 273 B1**
(45) Date of publication and mention of the grant of the patent: **27.01.2010**
(21) Application number: 05850345.9
(22) Date of filing: 10.11.2005
(51) Int. Cl.: C07C 227/32, C07C 227/18, C07C 229/22

(54) **METHOD FOR PREPARING DIASTEREOISOMERS OF 4-HYDROXY ISOLEUCINE**
VERFAHREN ZUR HERSTELLUNG VON DIASTEREOISOMEREN VON 4-HYDROXYISOLEUCIN
PROCÉDÉ DE PRÉPARATION DES DIASTÉRÉOISOMÈRES DE LA 4-HYDROXYISOLEUCINE

(30) Priority: 10.11.2004 EP 04292666
(43) Date of publication of application: 15.08.2007
(73) Proprietor: CENTRE NATIONAL DE LA RECHERCHE SCIENTIFIQUE (CNRS), 75794 Paris Cedex 16 (FR); Université Louis Pasteur Strasbourg I, 67000 Strasbourg (FR)
(72) Inventor: MIOSKOWSKI, Charles, F-67200 Strasbourg (FR); WAGNER, Alain, F-67000 Strasbourg (FR)
(74) Representative: Peaucelle, Chantal
(86) International application number: PCT/EP2005/013975
(87) International publication number: WO 2006/051000

(56) References cited:
- WO-A1-97/32577
- WO-A2-01/72688
- A. CORDOVA ET AL: "A Highly Enantioselective Amino Acid-Catalyzed Route to Functionalized alpha-Amino Acids" J. AM. CHEM. ASOC., vol. 124, no. 9, 2002, pages 1842-1843, XP002329293

## Description

The invention relates to a method for preparing diastereoisomers of 4-hydroxy isoleucine.

4-hydroxy isoleucine has the following structure

COOH- C(NH₂)- CH(CH₃) -CH(OH)-CH₃ (A)

Due to the presence of 3 chiral carbons, 4-hydroxy isoleucine will exist in eight isomeric forms, i.e.

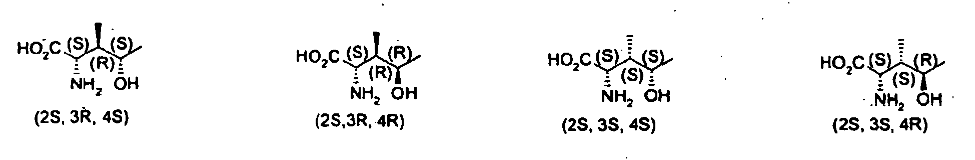

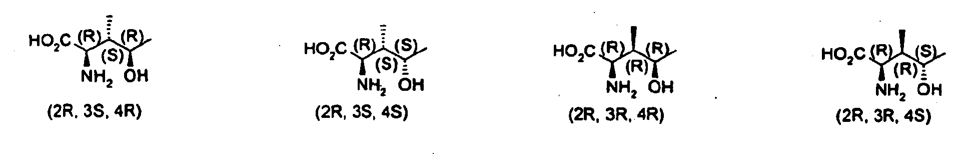

The inventors have developed a method to obtain any one of said isomers, as desired, using the same general reaction scheme, but changing specific condition reactions.

The method of the invention for preparing diastereoisomers, of 4-hydroxy isoleucine, of general formula A

CO₂H- CH(NH₂)- CH(CH₃)-CH(OH)-CH₃ (A)

comprising
deprotection of amine of Formula B 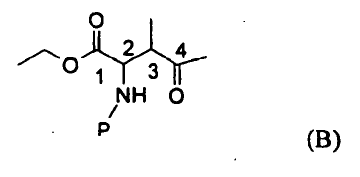
- reduction and subsequent lactonisation of an intermediate amine derivative of formula C 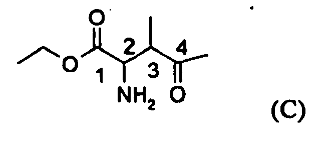 whose substituents at positions 2 and 3 are either R, S or S, S ; or S, R or R, R,
- hydrolysis of the resulting lactone of formula D 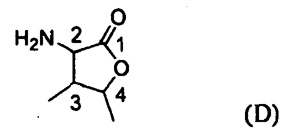
where in substituents at positions 2, 3 and 4 are
- S,R,S or S,R,R when using a protected amine S,R
- S,S,S or S,S,R when using a protected amine S,S
- R,S,R or R,S,S, when using a protected amine R,S
- R,R,R or R,R,S when using a protected amine R,R
under conditions to obtain the desired isomer of 4-hydroxy isoleucine.

Methods for the preparation of (2S, 3R, 4S)-4-hydroxy isoleucine have been disclosed in WO -A- 01/72688.

WO-A-97/32577 discloses the separation of the diastereisomers of the compound of formula (A) by extraction of Mediterraneum leguminous plants A. cordova et al. J. Am. Chem. Soc. 124 (9) , 2002.pp 1842- 1843 disclose the preparation of the coumpound of formula (B)

The invention particularly relates to a method, wherein according to a first embodiment said lactone is obtained in one pot, by oxidative deprotection (step 1) and borohydride reduction (step 2).

In another embodiment, in order to obtain other isomers, said lactone is obtained in two pots by deprotecting the amine with an oxidising agent resulting in an intermediate of formula C which is isolated. 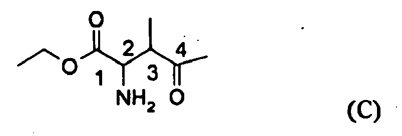 said intermediate derivative C being then submitted to a borohydride reduction to give the desired isomers.

Advantageously, said oxidizing agent is a mild oxidative agent.

Said first embodiment comprises the use of protected amine of formula B, having S, R substituents, respectively at positions 2 and 3, or having S, S substituents, respectively at positions 2 and 3.

Said second embodiment comprises the use of protected amine formula B having R, S substituents, respectively at positions 2 and 3, or R, R substituents, respectively at positions 2 and 3.

Said protected amine R, S or S, R of formula B is more preferably obtained by epimerisation of protected amine S, S or R, R, respectively.

Said epimerisation is advantageously carried out with DBN/t-BuOMe

Preferably, the protected amines (R, S, or S, R; S, S or R, R) of formula B are obtained by condensing a derivative of formula E 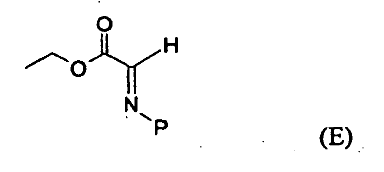 wherein P is an amino-protecting group, such as para-phenylmethoxy group, with 2-butanone 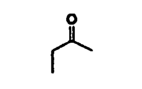 in the presence of proline.

In order to obtain said protected amines S, R, or S, S, L- proline is added to the reaction mixture.

In order to obtain said protected amines R, S, or R, R, D- proline is used instead of L-proline.

In a preferred embodiment of the invention said derivative of formula E is obtained by condensing an amino derivative of formula F

P-NH₂

wherein P is such as above defined,
with ethylglyoxalate of Formula G

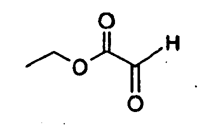

A particularly preferred method for preparing diastereoisomers of 4-hydroxy isoleucine comprises:
- a) condensing an anisidine derivative of formula I 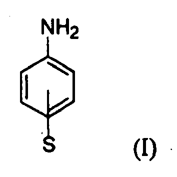 wherein S represents one or several substituents identical or different selected in the group comprising (C1 - C3) alkyl, alkoxy with a (C1 - C3) alkyl group or aryloxy, halogen such as F, Cl, Br, I, nitro and nitriles.
   with ethylglyoxalate of formula II 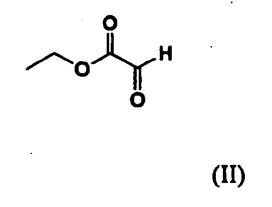 to give imine of formula III 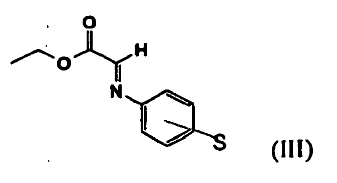
- b) condensing imine of formula III with 2-butanone IV 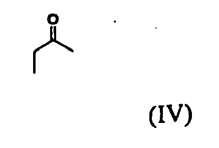 to give a protected amine of formula Va or Vb, 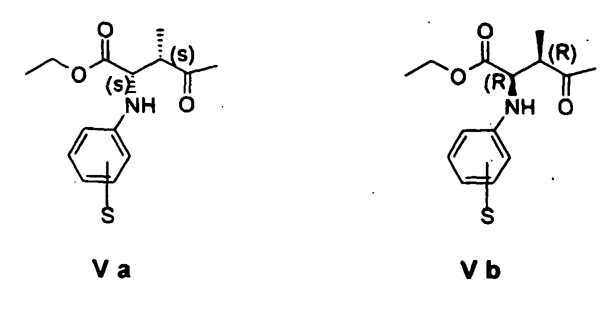 and if desired,
- c) epimerisation of the resulting condensation derivative of formula Va or Vb to give N-protected amine of formula VIa or VIb, respectively, 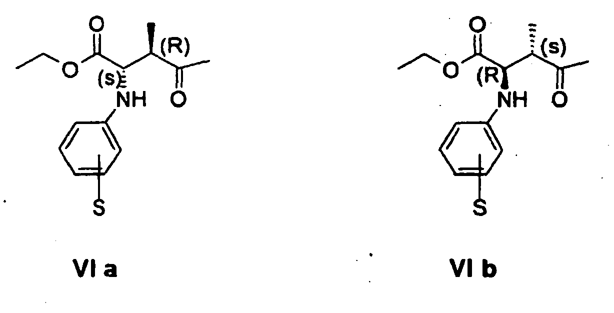
- d) reacting said N-protected amine Va or Vb, or VIa or VIb, under conditions to give -
   - lactone a 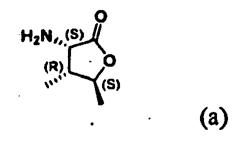 or
   - lactone b 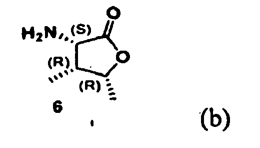 when using N-protected amine VIa
   - lactone c 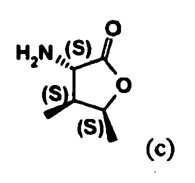 or
   - lactone d 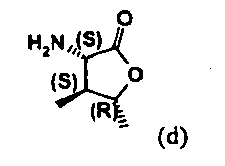 when using N-protected amine Va
   - lactone e 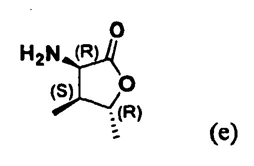 or
   - lactone f 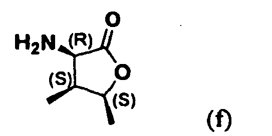 when using N-protected amine VIb
   - lactone g 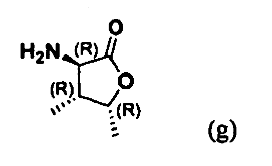 or
   - lactone h 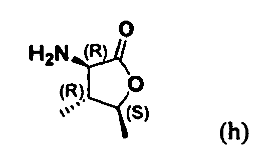 when using N-protected amine Vb
- e) hydrolysing said lactones to obtain the desired stereo isomer.

Step a) is advantageously carried out in an organic solvent such as toluene and all aromatic hydrocarbons, chlorosolvents such as chloroform, dichloromethane, polar protic and aprotic solvents such as DMF, DMSO, acetonitrile, dioxane, ether, THF. In a preferred embodiment, ethylglyoxalate of formula II is added to a solution of the p-anisidine derivative of formula I in said solvent, advantageously containing dehydrating agents, such as Na₂SO₄, MgSO₄, K₂SO₄, CaCl₂ molecular sieves and azeotropic distillation using aromatic hydrocarbon solvents like toluene, xylene, benzene.

The condensation of imine of formula III with 2-butanone according to step b) is advantageously carried out in the presence of an organo catalyst with both antipodes of pyrrolidine-2-carboxylic acid and its derivatives, preferably proline in an organic solvent or mixture of organic solvents, such as DMF, alcohols C1-C7, toluene or aromatic hydrocarbons, chloro solvents such as chloroform, dichloromethane, polar protic and aprotic solvents such as DMSO, acetonitrile, dioxane, ether, THF.

Both antipodes of pyrrolidine -2-carboxylic acid and its derivatives means, for example,

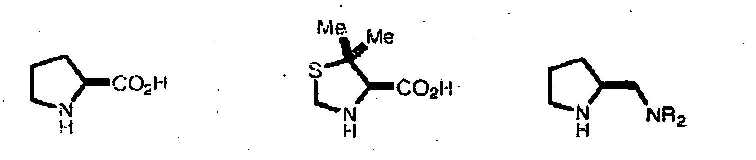

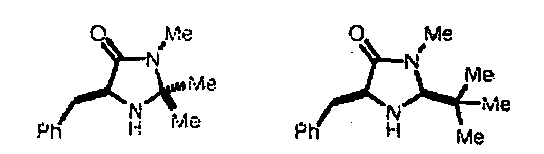

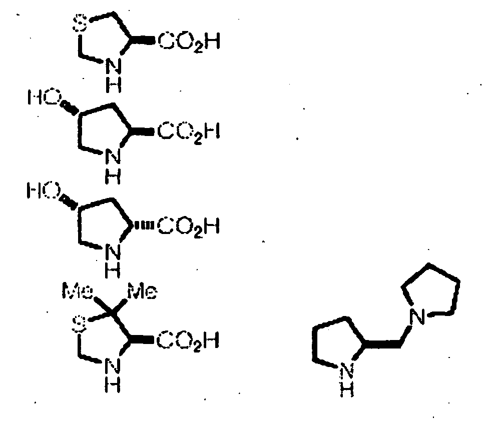

According to step c), epimerisation of the N-protected amine of formula Va or Vb is performed, by adding catalytic amounts of DBN, DBU, DABCO, DEIPA, TEA, Alkyl amines, alkali and alkaline, earth metal alkoxides, aryl amines, and substituted aryl amines, in solvents such as ethers, substituted ethers C1-C7 aromatic hydrocarbons and alcohols C1-C6, to a solution of the N-protected amine in a solvent such as t-BuOMe.

Step d) comprises the obtention of the lactones from the protected amines (VIa or VIb, Va or Vb).

According to a first embodiment, in order to prepare (2S, 3R, 4S) 4-hydroxy isoleucine isomer, step d) is carried out in one pot by reacting protected amine VIa in solution in an organic solvent, with an oxidising reagent at low temperature of- 4°C to + 4°C, preferably of about 0°C, neutralizing the reaction mixture at pH 7, adding a borohydride, reducing agent and adding a hydrolysing, to give resulting lactone a reaction mixture.

(2S, 3R, 4S) 4-hydroxy isoleucine isomer is recovered after acidification of the reaction mixture, evaporation of the solvents and crystallization.

Said oxidizing agent is advantageously selected in the group comprising Ceric Ammonium Nitrate (CAN), ammonium persulphate, sodium persulphate, potasium persulphate, transition metal oxides and other mild oxidising agents such as lithium perchlorate, sodium hypochlorate.

When the oxidizing agent does not comprise Cerium, said element is further added prior to the reduction.

Said borohydride reducing agent is preferably a metal borohydride of formula M(BH4)n, wherein M is selected in the group comprising sodium, lithium, magnesium, calcium, aluminium, tin, titanium, and said hydrolysing agent is preferably a metal hydroxide such as lithium hydroxide or sodium hydroxide.

According to a second embodiment, in order to prepare (2S, 3R, 4R), 4-hydroxy isoleucine isomer, step d) is carried out in two pots by reacting protected amine VIa with an oxidising agent such as above defined, neutralizing the reaction mixture at about pH 8, and recovering pure amine of formula VII.

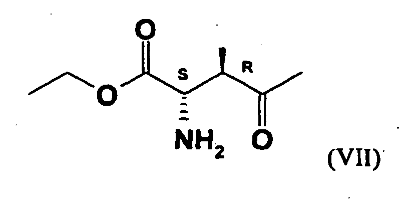

Said amine is then treated with a borohydride reducing agent such as above defined and an hydrolysing agent such as above defined, is added to the resulting lactone b reaction mixture.

(2S, 3R, 4S) 4-hydroxy isoleucine isomer is recovered after acidification of the reaction mixture, evaporation of the solvents and crystallization.

According to a third embodiment, in order to prepare (2S, 3S, 4S), 4-hydroxy isoleucine isomer, step d) is carried out in one pot, as above defined with respect to step c) except that protected amine Va is used instead of protected amine VIa. (2S, 3S, 4S) 4-hydroxy isoleucine isomer is recovered after acidification of the reaction mixture, evaporation of the solvents and crystallization.

According to a fourth embodiment, in order to prepare (2S, 3S, 4R), 4-hydroxy isoleucine isomer, step d) is carried out in two pots, as above defined with respect to the obtention of (2S, 3R, 4R), except that protected amine Va is used instead of protected amine VIa.

(2S, 3S, 4R) 4-hydroxy isoleucine isomer is recovered after acidification of the reaction mixture, evaporation of the solvents and crystallization.

By using the same protocols as above defined with respect to the obtention of (2S, 3R, 4S), (2S, 3R, 4R), (2S, 3S, 4S) and (2S, 3S, 4R), but using protected amines VIb or Vb, respectively, the following isomers are obtained:
(2R, 3S, 4R), 4-hydroxy isoleucine isomer,
(2R, 3S, 4S), 4-hydroxy isoleucine isomer,
(2R, 3R, 4R), 4-hydroxy isoleucine isomer, and
(2R, 3R, 4S), 4-hydroxy isoleucine isomer.

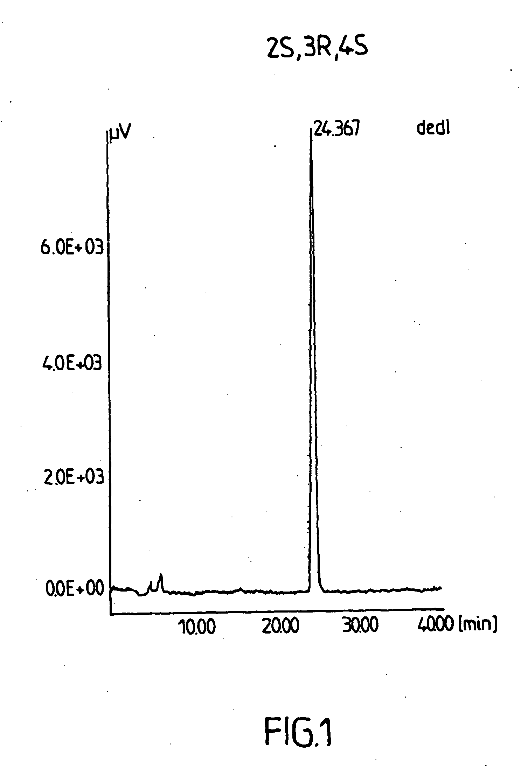

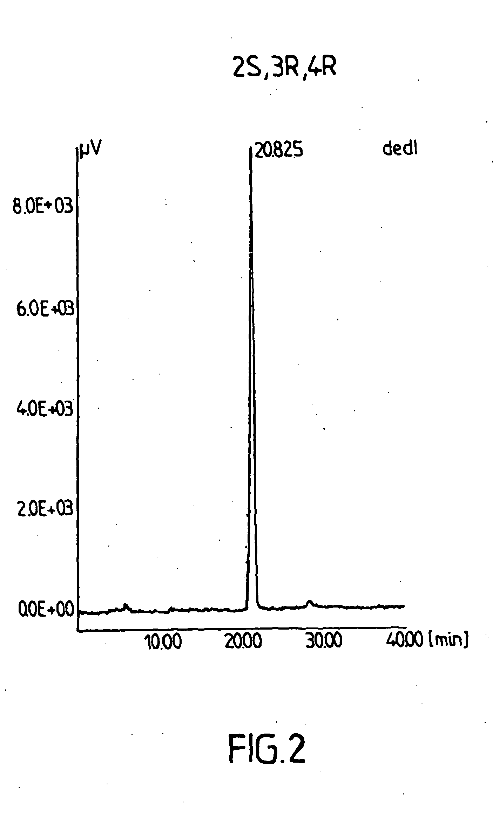

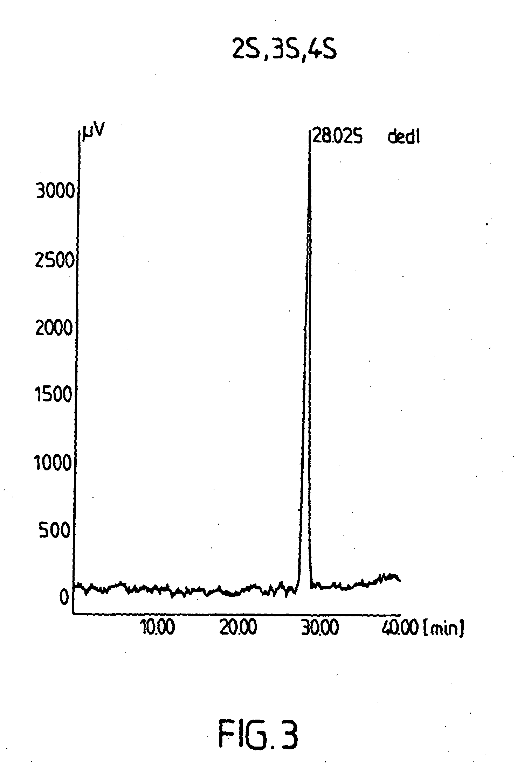

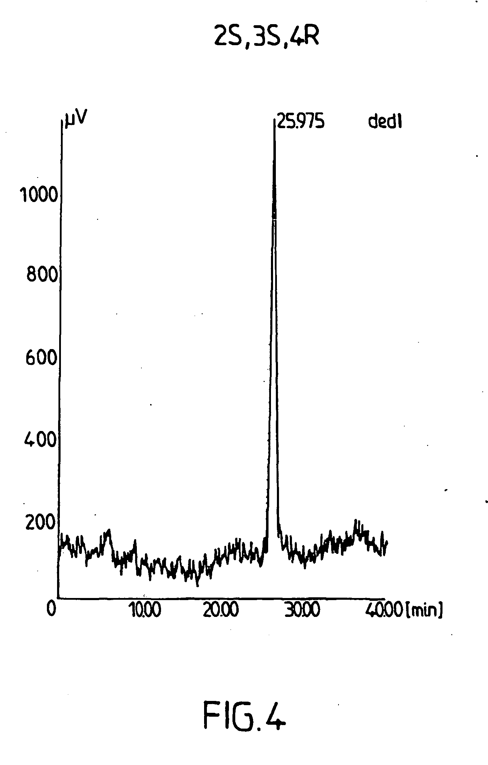

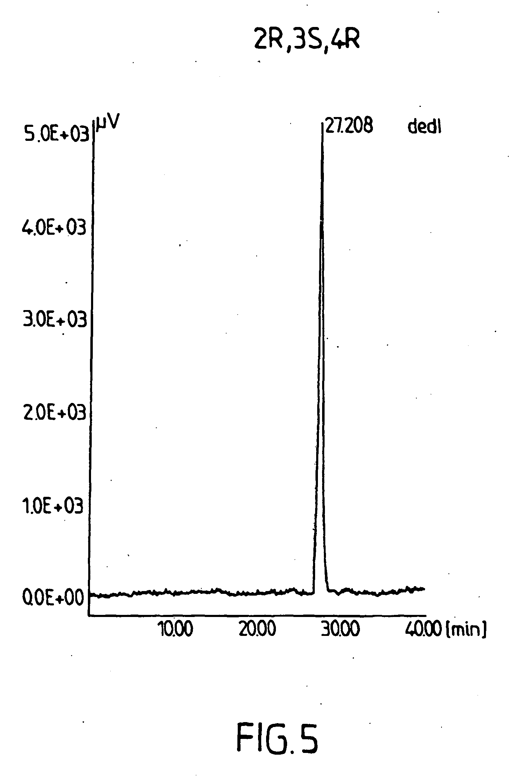

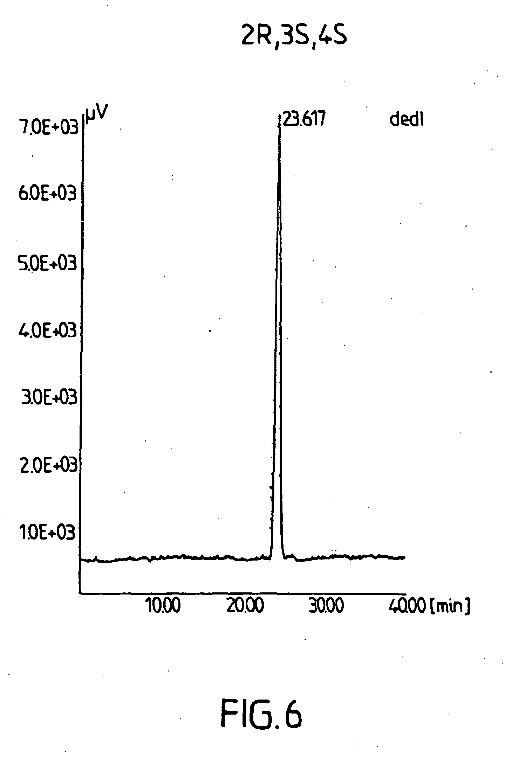

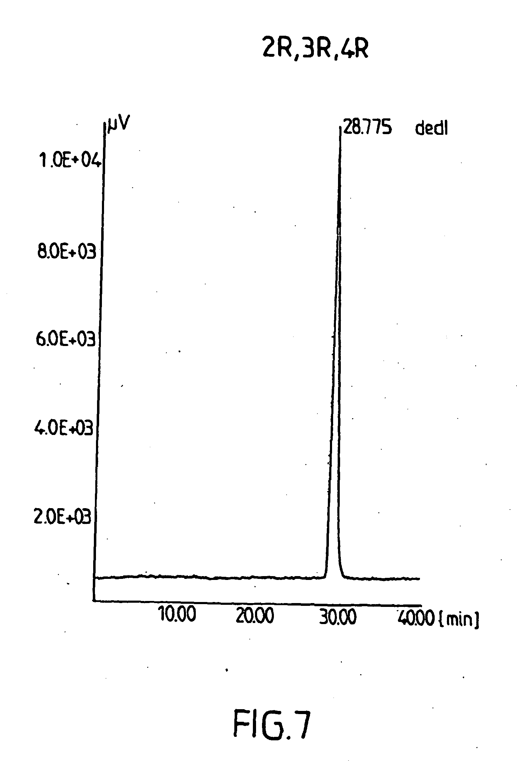

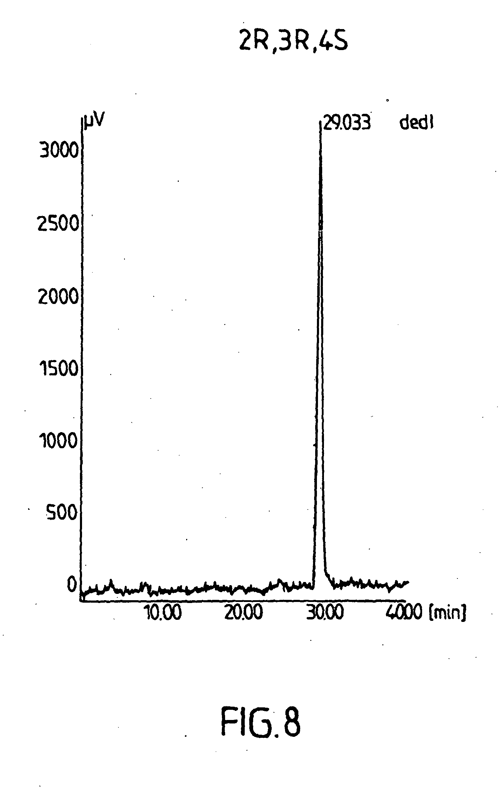

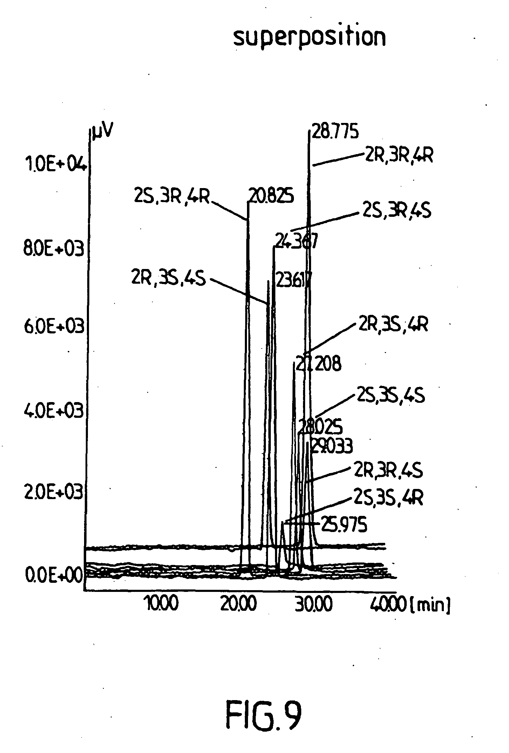

Other characteristics and advantages of the invention will be given hereinafter with reference to figures 1 to 8, which represent, the HPLC of the 8 stereo isomers, respectively. The analysis was performed on a Chirobiotic column with 0.5 mL / mL flow, using CH₃CN/H₂O 75/25 at 25° C. DEDL detector was used at 50° C / gain 7/P 3.4 bars Helium, and figure 9 which shows superposition of HPLC curves of the 8 isomers of 4-hydroxy Isoleucine.

### Example 1: Preparation of N-pmp amine of formula (5)

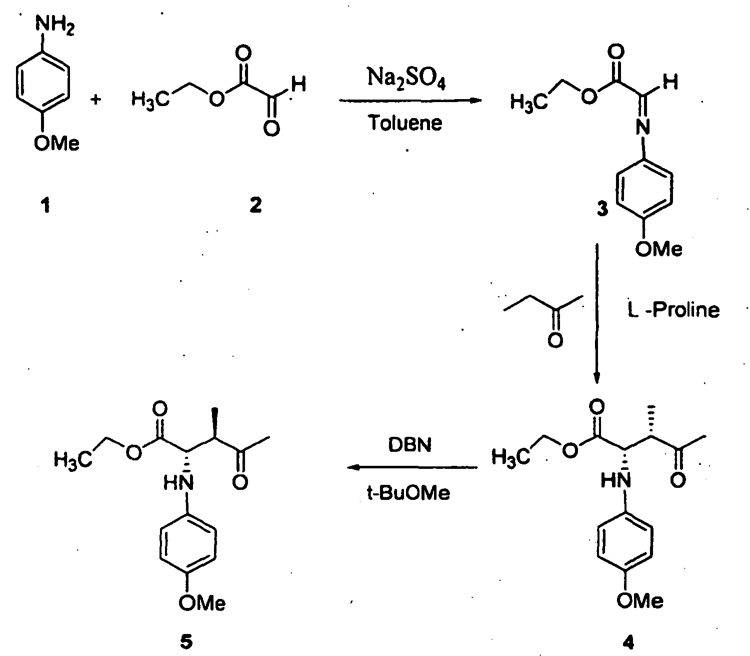

### preparation of imine (3) :

50 g of p-anisidine **(1)** (406 mmol) was dissolved in 400 mL of toluene and 200 g of sodium sulfate (aprox: 2.5 eq) was added and stirred. 82 mL of ethyl glyoxalate **(2)** (50% in toluene 1 eq) was added slowly and proper stirring was maintained. The reaction was completed in 30 min. Sodium sulfate was filtered off using Celite® and toluene was removed under vacuum. 80 g (95% yield) of crude product **(3)** was recovered. The next condensation reaction was carried out on the crude reaction product.

### Synthesis of N-PMP amine (5):

2-butanone (800 mL, 22 eq), dry DMF (600 mL) and L- Proline (15.8 gm, 0.35 eq) were added and stirred at room temperature under nitrogen.

Imine **(3)** obtained in the above reaction was dissolved in 200 mL of dry DMF along with Et₃N (0.40 eq 22.4 mL) and added slowly to the reaction mixture (L-Proline + butanone + DMF) under nitrogen and maintained under stirring during about 8 h, L-Proline, butanone and DMF were then successively removed. After complete removal of DMF, the crude mas s **(4)** (red colored gum) was dissolved in 15 mL of t-BuOMe and 1 mL of DBN (catalytic 0.04 eq) was added and stirred under nitrogen for 2 hrs. The t-BuOMe was allowed to evaporate in overnight at room temperature. After the isomerisation, a solid cake was obtained, dissolved in hot ethanol and crystallized. 48 gms of pure isomerised product **(5)** were recovered (Total yield: 43%)

### Example 2: Synthesis of (2S, 3R, 4S)- 4-hydroxy Isoleucine:

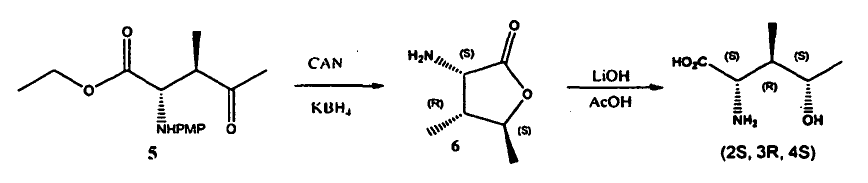

11.6 g (40 mmol) of N-pmp protected amine **(5)** was dissolved in 20 mL of acetonitrile. CAN (65.6 g, 3 eq) in 120 mL of water was added with stirring and the reaction temperature inside the flask maintained at 0°C. The initially blue reaction mixture gradually changed to green after complete addition of CAN. The reaction mixture was stirred for 2 h 30 min then extracted with ethylacetate (4 x 150 mL) and the aqueous phase is preserved for the next reduction reaction.

The aqueous phase was neutralised with saturated sodium carbonate and pH adjusted to 7. After neutralisation of the reaction mixture, the reaction flask was cooled to -15°C with proper stirring and maintained for 30 min. Potassium borohydride (3.2 g, 60 mmol, 1.5 eq) was then added to the reaction mixture.

The reaction was allowed to come to 0°C for about 45 min and then basified with 2N Sodium carbonate to pH around 8-9 and extracted with methylene dichloride (5 x 400 mL). The combined organic phases were washed with water, dried over sodium sulfate and evaporated under reduced pressure. 3.73 g (62.6% yield) of the resulting lactone mixture **(6)** was recovered.

The lactone mixture **(6)** was dissolved in 96 mL of water (0.3 M) and 1.1g (43.3 mmol 1.5 eq) of LiOH was added and stirred at room temperature for 2 h. The reaction mixture was carefully acidified with AcOH (43.3 mmol, 2.4 mL). The aqueous phase in the reaction mixture was evaporated in vacuum and ethanol was added and evaporated till all the water was evaporated. The gummy material obtained was crystallised from absolute ethanol.
yield: 1.56 g of Isomer (2S, 3R, 4S) 4-hydroxy Isoleucine was obtained (purity: 98%)
**(HPLC).**
The isomer was further purified by preparative HPLC to get white shine powder.

**¹H NMR** (200 MHz, D₂O): δ 3.90 (m, 1H), 3.84 (m, 1H), 1.91 (m, 1H), 1.23 (d, *J* = 5.6 Hz, 3H) 0.95 (d, *J*= 6.6 Hz, 3H)
**¹³C NMR** (75 MHz, D₂O): δ 174.32, 70.46, 57.54, 41.90, 21.30, 12.70.

### Example 3: Synthesis of (2S, 3R, 4R)-4- hydroxy Isoleucine:

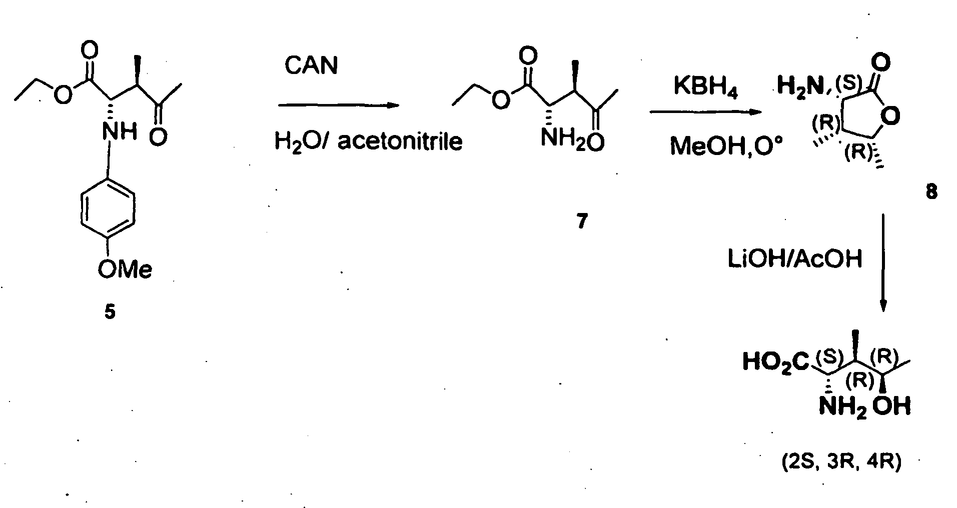

The same procedure as defined in example 2 is applied except that the reaction mixture with CAN is stirred for 45 min, instead of 2 h 30 min. and the aqueous phase obtained after extraction with ethylacetate is neutralized with saturated sodium bicarbonate solution and made slightly basic (pH is maintained around 8). The aqueous phase is then extracted with 4 x 150 mL of dichloromethane. The combined organic extracts were washed with brine, dried over anhydrous sodium sulfate and concentrated under vacuum to yield 5:52 g (79.7%) of pure amine **(7)** as brown coloured oil,

The above-obtained amine **(7)** was dissolved in 15 mL of methanol and cooled to 0° C and 2.58 g (47.8 mmol) of potassium borohydride solid was added in one time. The reaction mixture is stirred at 0° C for 45 min. and gradually brought to room temperature. Methanol is removed from the reaction mixture under reduced pressure.

The aqueous phase was extracted with 4 x 150 mL of dichloromethane. The combined organic phases were washed with brine, dried and evaporated in vacuum to give 2.9 g (70.2%) of lactone mixture **(8)** (75:25).

The lactone crude **(8)** was taken up for further reaction without any further purification. The lactone mixture was dissolved in 100 mL of water and 805 mg (33.7 mmol) of LiOH added. The reaction was maintained at room temperature for 1 h and acidified with AcOH (1.91 mL, 33.72 mmol). The water in the reaction mixture was removed under reduced pressure and the crude gummy mass was dissolved in absolute ethanol and again ethyl alcohol was removed. The crude gummy mass thus obtained was dissolved in 90% ethanol and cooled. The separated solid was filtered and washed with ethanol. The grey coloured solid was crystallized from 90% ethanol and yielded 1.4 g (75:25). The sample was further purified with preparative HPLC to get pure white shine material.

**¹H NMR** (300 MHz, D₂O): δ4.05 (m, 1H), 3.80 (d, *J=* 4.2 Hz1H), 2.13 (m, 1H) 1.20 (d,*J*= 6.3 Hz. 3H), 1.05 (d, *J* = 7.2 Hz, 3H)
**¹³C NMR** (75 MHz, D₂O): δ174.49, 69.13, 59.97, 39.12, 20.71, 9.38

### Example 4:Synthesis of (2S,3S,4S)-4- hydroxy Isoleucine:

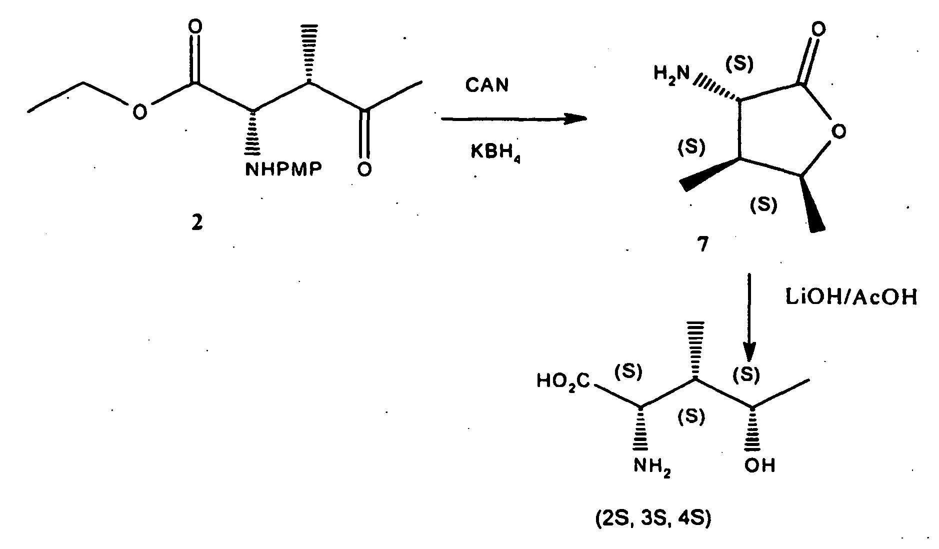

The Mannich condensation product **(2)** was purified by column chromatography to remove regio isomer and p-anisidine using Hex: EtOAc (85:15) as eluent.

5.6 g (20 mmol) of N-pmp protected amine**(2)** was taken in 250 mL round bottomed flask and was dissolved in 10 mL of acetonitrile. Ceric Ammonium nitrate (CAN) (33 g, 3 eq) in 60 mL of water was added with stirring and maintained the reaction temperature inside the flask at 0°C. The reaction mixture was initially blue in colour and gradually changed to green after complete addition of CAN. The reaction mixture was stirred for 45 min and checked by TLC (hex: EtOAc 7:3) for the presence of starting material. The reaction mixture was extracted with ethylacetate (4 x 150 mL). The aqueous phase was neutralised with saturated sodium carbonate and adjusted to pH 7. After neutralisation of the reaction mixture, the reaction flask was cooled to -15°C with proper stirring. After cooling was maintained for 30 min., 1.6 g of potassium borohydride (30 mmol, 1.5 eq) was added to the reaction mixture. The reaction was allowed to come to 0°C in for about 45 min. The reaction mixture was basified further with 2 N sodium carbonate to the pH around 8-9 and extracted with methylene dichloride (5 x 400 mL). The combined organic phases were washed with water, dried over sodiumsulfate and evaporated under reduced pressure 1.42 g of lactone mixture was obtained.

The crude lactone mixture **(7)** was dissolved in 35 mL of water and 395 mg (16.5 mmol, 1.5 eq) of LiOH was added and stirred at room temperature for two hrs. Tthe reaction mixture is carefully acidified with AcOH (16.5 mmol, 0.9 mL). The aqueous phase in the reaction mixture was evaporated in vacuum and ethanol was added and evaporated till all the water was evaporated. The gummy material obtained was dissolved in 90% ethanol and left overnight. The separated white solid flakes were filtered and washed several times with ethanol to remove lithium acetate impurities. The crude solid was recrystallized with 90% ethanol to give white crystals of (2S, 3S, 4S)-4- hydroxy Isoleucine, 500 mg.
The sample is further purified by preparative HPLC to get white shine powder.

**¹H NMR** (300 MHz, D₂O): δ4.11 (m, 1H), 3.87 (d,*J* = 2.7Hz 1H), 2.21 (m, 1H), 1.23 (d, *J*= 6.3 Hz, 3H), 0.92 (d, *J*= 7.5 Hz, 3H)
**¹³C NMR** (75 MHz, D₂O): δ174.64, 71.39, 60.39, 38.97, 21.11, 6.19

### Example 5: Synthesis of (2S,3S,4R) -4-hydroxy Isoleucine: MSK-4)

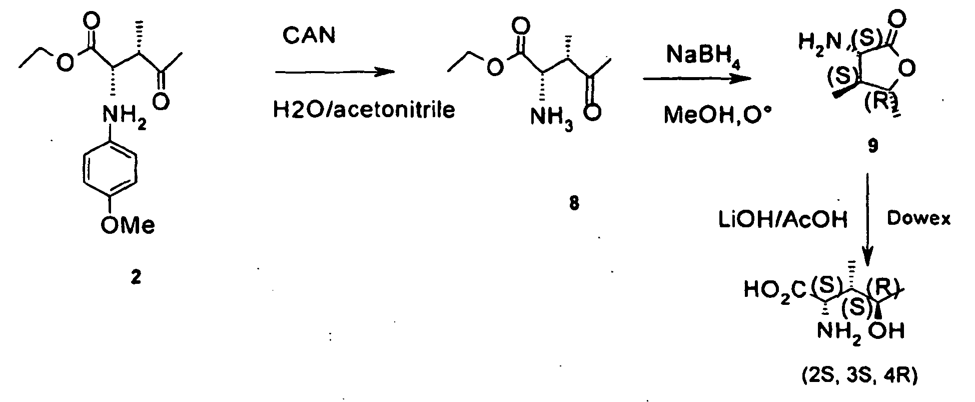

The reaction was carried out as mentioned above for (2S, 3R, 4R) isomer and resulted in pure amine **(8)** as brown coloured oil.

The above-obtained amine **(8)** was dissolved in 15 mL of methanol and cooled to 0° C and 962 mg (1.1 eq, 25.43 mmol) of sodium borohydride solid was added in one time. The reaction mixture was stirred well at 0°C for 45 min. and gradually brought to room temperature. Methanol was removed from the reaction mixture and diluted with water. The aqueous phase was extracted with 4 x 150 mL of dichloromethane. The combined organic phases were washed with brines, dried and evaporated to give 2 g of complex mixture of lactones.

The lactone crude (15.5 mmol) **(9)** was taken up for further reaction without any further purification. The lactone mixture was dissolved in 40 mL of water and to this 556.9 mg (18.6 mmol) of LiOH added. The reaction was maintained at room temperature for 1 h and is acidified with AcOH (1.31 mL). The water in the reaction mixture was removed under reduced pressure and the crude gummy mass was dissolved in absolute ethanol and said ethanol was further removed. The crude gummy mass thus obtained was dissolved in minimum amount of water and the compound was loaded on a column packed with dowex50wx8 (H+) resine (50 g). The column was first eluted with water 4 x 50 mL and then fractions were collected by eluting with 2M ammoniumhydroxide. The ninhydrine positive fractions were combined and evaporated in vaccum to get crude solid free from lithium acetate. The crude solid was dissolved in 90% ethyl alcohol and kept over night. The separated solid (250 mg) was filtered and washed with cold ethyl alcohol and recrystallized from 90% ethanol.

This diastereomer mixture was purified by preparative HPLC to get pure (2S, 3S, 4R) isomer as a white shine powder.

**¹H NMR** (300 MHz, D₂O): δ4.02 (d, *J =* 3Hz, 1H), 3.81 (m, 1H), 2.12 (m, 1H) 1.28 (d, *J* = 6.6 Hz, 3H), 0.97 (d, *J* = 7.2 Hz, 3H)
**¹³C NMR** (75 MHz, D₂O): δ174.93, 70.18, 56.34, 40.46, 21.24, 12.15

**Examples 6: Synthesis of enantiomers:** The same strategy was employed for the synthesis of enantiomers of above described molecules, except that D-Proline was used instead of L-proline in Mannich condensation reaction to obtain the enantiomer.
**(2R, 3S, 4R)- 4-hydroxy Isoleucine**
**¹H NMR** (200 MHz, D₂O): δ3.89 (m, 1H), 3.84 (m, 1H), 1.90 (m, 1H) 1.23 (d, *J* = 6.4 Hz, 3H) 0.95 (d, *J*= 7 Hz, 3H)
**¹³C NMR** (50 MHz, D₂O): δ174.36, 70.43, 57.51, 41.91, 21.30, 12.6
**(2R, 3S, 4S) 4-hydroxy Isoleucine**
**¹H NMR** (200 MHz, D₂O): δ4.04 (m, 1H), 3.80 (m, 1H), 2.12 (m, 1H), 1.19 (d, *J* = 6.2 Hz, 3H) 1.05 (d, *J*= 7.2 Hz, 3H)
**¹³C NMR** (50 MHz, D₂O): δ 174.55, 69.12, 59.97, 39.12, 20.73, 9.40
**(2R, 3R, 4R) 4-hydroxy Isoleucine**
**¹H NMR** (200 MHz, D₂O): δ4.10 (m, 1H), 3.87 (d, *J* = 2.6 Hz 1H), 2.23 (m, 1H) 1.23 (d, *J* = 6.6 Hz, 3H), 0.92 (d, *J* = 7.2 Hz, 3H)
**¹³C NMR** (50 MHz, D₂O): δ174.64, 71.29, 60.35, 38.96, 21.12, 6.22
**(2R, 3R, 4S) 4-hydroxy Isoleucine**
**¹H NMR** (300 MHz, D₂O): δ4.01 (d, *J* = 2.7 Hz, 1H), 3.80 (m, 1H), 2.11 (m, 1H) 1.27 (d, *J* = 6.3 Hz, 3H), 0.97 (d, *J* = 7.2 Hz, 3H)
**¹³C NMR** (75 MHz, D₂O): δ 174.96, 70.18, 56.35, 40.44, 21.23, 12.10

| **Isomers of 4-HIL *** | **[α]²¹_{D} in H₂O** | **m.p** |
|---|---|---|
| **2S, 3R, 4S** | (+) 30.7 c, 1 | 215-22 ° C (subl.) |
| **2S, 3R, 4R** | (-) 21.6 c, 0.5 | 202-04 ° C (subl.) |
| **2S, 3S, 4S** | (+) 28.0 c, 0.25 | 253-55 ° C (dec.) |
| **2S, 3S, 4R** | (+) 6.0 c, 0.25 | 173-75 ° C |
| **2R, 3S, 4R** | (-) 31.0 c, 1 | 217-25 °C (subl.) |
| **2R, 3S, 4S** | (+) 22.0 c, 0.5 | 200-04 °C (subl.) |
| **2R, 3R, 4R** | (-) 30.0 c, 0.25 | 250-54 °C |
| **2R,3R,4S** | (-) 5.6 c, 0.25 | 173°C |

| | | |
|---|---|---|
| **(* HIL = hydroxy Isoleucine)** | | |

### DEPROTECTION OF PMP (para-methoxyphenyl-amines)

4.9 gm (0.02mol) ammonium persulphate, 0.29 gm (0.001 mol) of CAN was taken in 40 ml of water.
3 gm (0.01 mol) of PMP protected amine in 6 ml of acetonitrile was added to the reaction mixture slowly.
The contents are stirred for about 2 hrs at 35 - 40 °C.
After ensuring the absence of starting material on TLC, the reaction mixture was extracted with ethyl acetate (100 ml x 5).
The aqueous layer was basified to pH 8-9 using sodium bicarbonate solution.
The aqueous layer was extracted with dichloromethane.
The organic layer was dried with an. Na₂SO₄, and concentrated.
Reddish brown colored liquid (1 g) was obtained.
Yield 70 %

## Claims

1. A method for preparing diastereoisomers of 4-hydroxy isoleucine of formula A
CO₂H- CH(NH₂)- CH(CH₃)-CH(OH)-CH₃ (A)
comprising
- deprotection of amine of Formula B 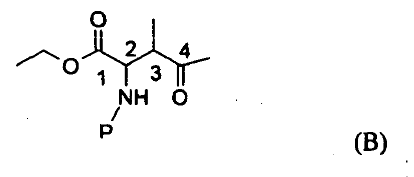
- reduction and subsequent lactonisation of an intermediate amine derivative of formula C 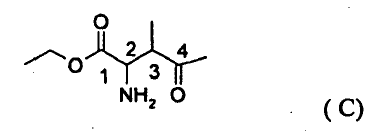 whose substituents at positions 2 and 3 are either R, S or S, S ; or S, R or R, R,
- hydrolysis of the resulting lactone of formula D 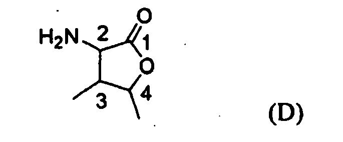 where in substituents at positions 2, 3 and 4 are
- S,R,S or S,R,R when using a protected amine S,R
- S,S,S or S,S,R when using a protected amine S,S
- R,S,R or R,S,S, when using a protected amine R,S
- R,R,R or R,R,S when using a protected amine R,R
under conditions to obtain the desired isomer of 4-hydroxy isoleucine.

2. The method of claim 1, wherein said lactone is obtained in one pot, by oxidative deprotection and borohydride reduction.

3. The method of claim 1, wherein said lactone is obtained in two pots by deprotecting the amine with an oxidising agent resulting in an intermediate of formula C which is isolated 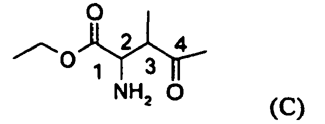 said intermediate derivative C being then submitted to a borohydride reduction reaction to give the desired isomers.

4. The method of anyone of the preceding claims, comprising using said protected amine of formula B, having R, S substituents, respectively at positions 2 and 3.

5. The method of anyone of claims 1 to 3, comprising using said protected amine of formula B, having S, S substituents, respectively at positions 2 and 3.

6. The method of anyone of claims I to 3, comprising using said protected amine of formula B, having R, R substituents, respectively at positions 2 and 3.

7. The method of anyone of claims 1 to 3, comprising using said protected amine of formula B, having S,R substituents, respectively at positions 2 and 3.

8. The method of any one of claims 1 to 7, wherein said protected amine R, S or S, R of formula B is obtained by epimerisation of the corresponding protected amine S, S or R, R.

9. The method of claim 8, wherein said epimerisation is carried out with DBN/t-BuOMe.

10. The method of anyone of claims 1 to 9, wherein the protected amines of formula B are obtained by condensing a derivative of formula E 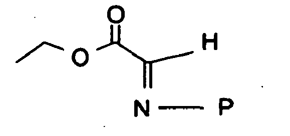 wherein P is an amino-protecting group,
with 2-butanone 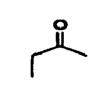 in the presence of proline.

11. The method of claim 10, wherein the proline is L- proline.

12. The method of claim 10, wherein the proline is D- proline.

13. The method of anyone of the preceding claims, wherein the derivative of formula E is obtained by condensing an amino derivative of formula F
P-NH₂ (F)
with ethylglyoxalate of formula G

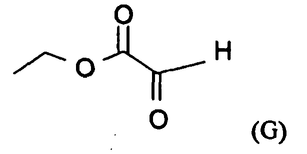

14. The method of anyone of the preceding claims for preparing diastereoisomers of 4-hydroxy isoleucine comprises:
- a) condensing an anisidine derivative of formula I 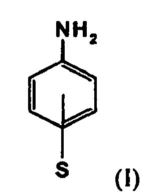 wherein S represents one or several substituents identical or different selected in the group conprising (C1 - C3) alkyl, alkoxy with a (C1 - C3) alkyl group or aryloxy, halogen such as F, Cl, Br, I, nitro and nitriles.
with ethylglyoxalate of formula II 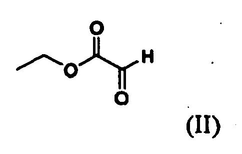 to give imine of formula III 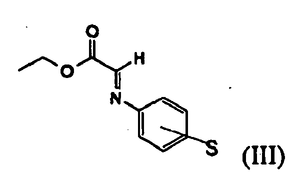
- b) condensing imine of formula III with 2-butanone IV 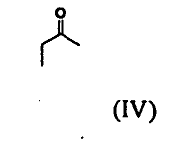 to give a protected amine of formula Va or Vb, 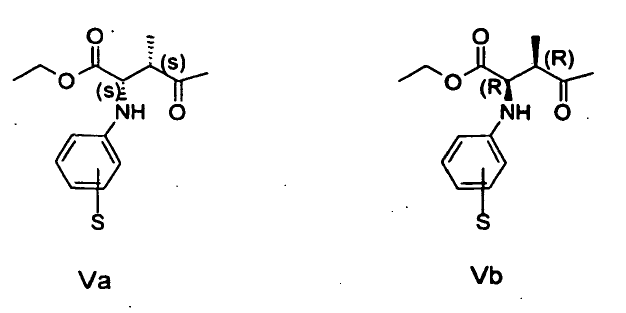 and if desired,
- c) epimerisation of the resulting condensation derivative of formula Va or Vb to give N-protected amine of formula VIa or VIb, respectively, 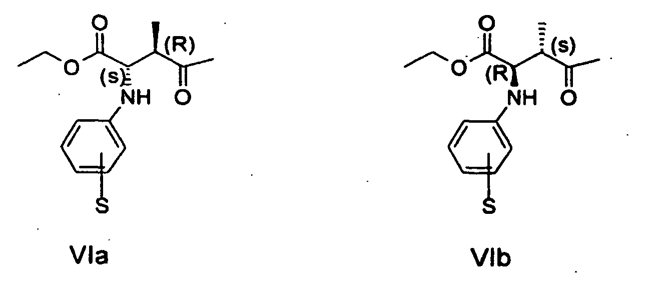
- d) reacting said N-protected amine Va or Vb, or VIa or VIb, under conditions to give
• lactone a 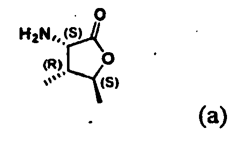 or
• lactone b 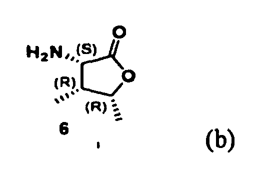 when using N-protected amine VIa
• lactone c 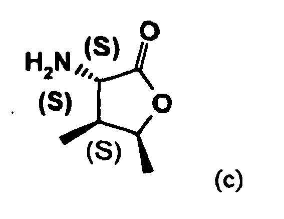 or
• lactone d 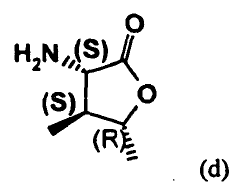 when using N-protected amine Va
or
• lactone e 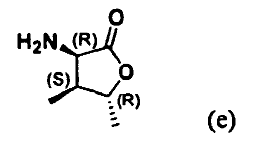 or
• lactone f 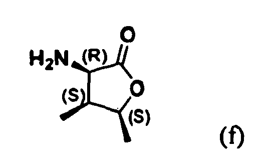 when using N-protected amine VIb
• lactone g 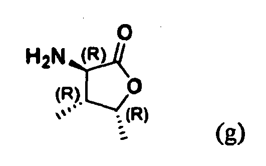 or
• lactone h 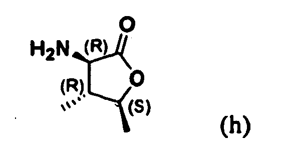 when using N-protected amine Vb
- e) hydrolysing said lactones to obtain the desired stereo-isomer.

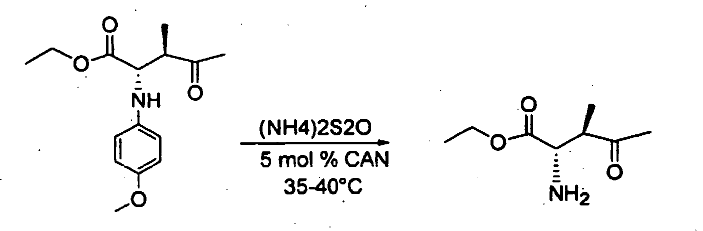

## Patentansprüche

1. Verfahren zur Herstellung von Diastereoisomeren von 4-Hydroxyisoleucin der Formel A
CO₂H- CH(NH₂)- CH(CH₃)-CH(OH)-CH₃ (A)
umfassend
- Entfernung der Schutzgruppen vom Amin der Formel B 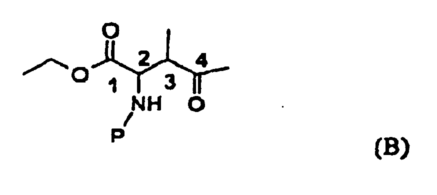
- Reduktion und anschließende Lactonisierung eines Aminderivat-Zwischenprodukts der Formel C 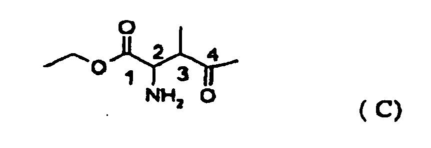 dessen Substituenten auf den Positionen 2 und 3 entweder R,S oder S,S oder S,R oder R,R sind;
- Hydrolyse des resultierenden Lactons der Formel D 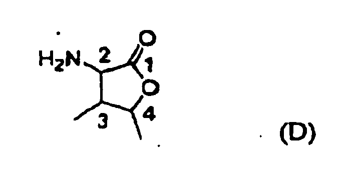 wobei die Substituenten auf den Positionen 2, 3 und 4
- S,R,S oder S,R,R sind, wenn man ein geschütztes S,R-Amin verwendet;
- S,S,S oder S,S,R sind, wenn man ein geschütztes S,S-Amin verwendet;
- R,S,R oder R,S,S sind, wenn man ein geschütztes R,S-Amin verwendet;
- R,R,R oder R,R,S sind, wenn man ein geschütztes R,R-Amin verwendet;
unter solchen Bedingungen, dass man das gewünschte Isomer von 4-Hydroxyisoleucin erhält.

2. Verfahren gemäß Anspruch 1, wobei das Lacton in einem Topf durch oxidative Entfernung der Schutzgruppen und Borhydrid-Reduktion erhalten wird.

3. Verfahren gemäß Anspruch 1, wobei das Lacton in zwei Töpfen durch Entfernung der Schutzgruppen des Amins mit einem Oxidationsmittel erhalten wird, was zu einer Zwischenstufe der Formel C 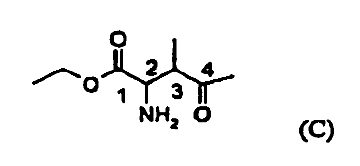 führt, die isoliert wird, wobei das Zwischenstufenderivat C dann einer Borhydrid-Reduktionsreaktion unter Bildung der gewünschten Isomere unterzogen wird.

4. Verfahren gemäß einem der vorstehenden Ansprüche, umfassend die Verwendung des geschützten Amins der Formel B, das R,S-Substituenten auf den Positionen 2 bzw. 3 aufweist.

5. Verfahren gemäß einem der Ansprüche 1 bis 3, umfassend die Verwendung des geschützten Amins der Formel B, das S,S-Substituenten auf den Positionen 2 bzw. 3 aufweiset.

6. Verfahren gemäß einem der Ansprüche 1 bis 3, umfassend die Verwendung des geschützten Amins der Formel B, das R,R-Substituenten auf den Positionen 2 bzw. 3 aufweist.

7. Verfahren gemäß einem der Ansprüche 1 bis 3, umfassend die Verwendung des geschützten Amins der Formel B, das S,R-Substituenten auf den Positionen 2 bzw. 3 aufweist.

8. Verfahren gemäß einem der Ansprüche 1 bis 7, wobei das geschützte R,S- oder S,R-Amin der Formel B durch Epimerisierung des entsprechenden geschützten S,S- oder R,R-Amins erhalten wird.

9. Verfahren gemäß Anspruch 8, wobei die Epimerisierung mit DBN/t-BuOMe durchgeführt wird.

10. Verfahren gemäß einem der Ansprüche 1 bis 9, wobei die geschützten Amine der Formel B durch Kondensieren eines Derivats der Formel E 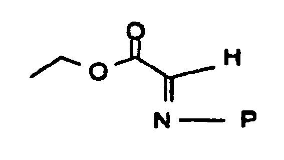 wobei P eine Aminoschutzgruppe ist, mit 2-Butanon 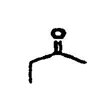 in Gegenwart von Prolin erhalten werden.

11. Verfahren gemäß Anspruch 10, wobei es sich bei dem Prolin um L-Prolin handelt.

12. Verfahren gemäß Anspruch 10, wobei es sich bei dem Prolin um D-Prolin handelt.

13. Verfahren gemäß einem der vorstehenden Ansprüche, wobei das Derivat der Formel E durch Kondensieren eines Aminoderivats der Formel F
P-NH₂ (F)
mit Ethylglyoxalat der Formel G 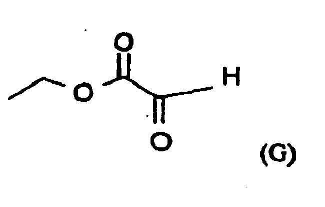 erhalten wird.

14. Verfahren gemäß einem der vorstehenden Ansprüche zur Herstellung von Diastereosiomeren von 4-Hydroxyisoleucin, umfassend:
- a) Kondensieren eines Anisidinderivats der Formel I 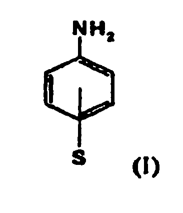 wobei S für einen oder mehrere Substituenten steht, die gleich oder verschieden sind und aus der Gruppe ausgewählt sind, die (C₁-C₃)-Alkyl, Alkoxy mit einer (C₁-C₃)-Alkylgruppe, oder Aryloxy, Halogen, wie F, Cl, Br, I, Nitro und Nitrile umfasst;
mit Ethylglyoxalat der Formel II 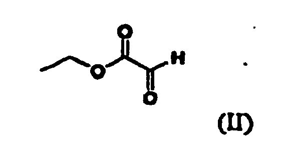 unter Bildung eines Imins der Formel III 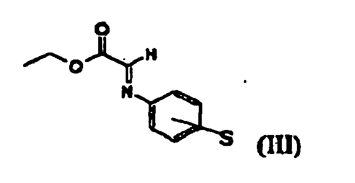
- b) Kondensieren des Imins der Formel III mit 2-Butanon IV 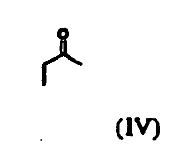 unter Bildung eines geschützten Amins der Formel Va oder Vb 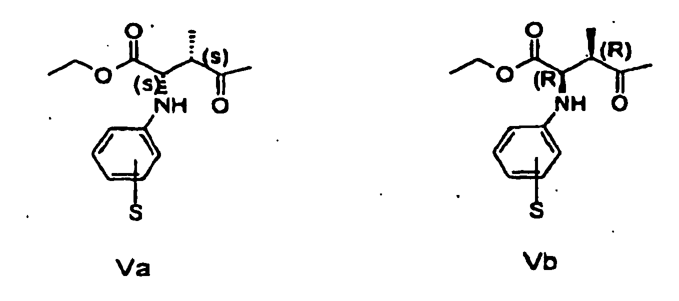 und gegebenenfalls
- c) Epimerisierung des resultierenden Kondensationsderivats der Formel Va oder Vb unter Bildung eines N-geschützten Amins der Formel VIa bzw. VIb 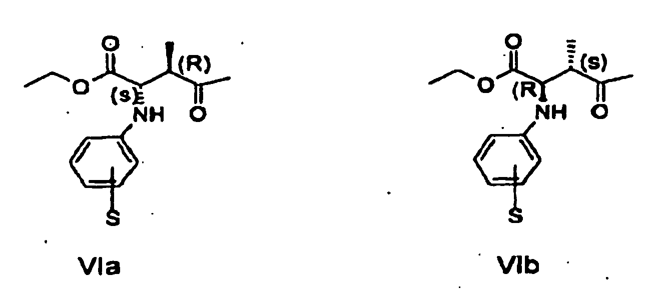
- d) Umsetzen des N-geschützten Amins Va oder Vb oder VIa oder VIb unter solchen Bedingungen, dass man
• Lacton a 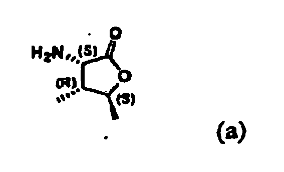 oder
• Lacton b 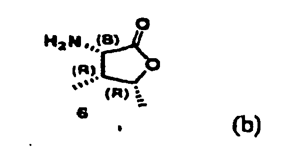 erhält, wenn man das N-geschützte Amin VIa verwendet;
• Lacton c 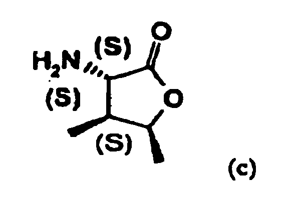 oder
• Lacton d 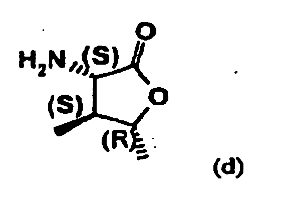 erhält, wenn man das N-geschützte Amin Va verwendet; oder
• Lacton e 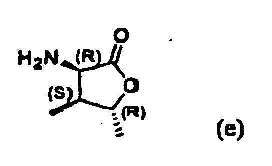 oder
• Lacton f 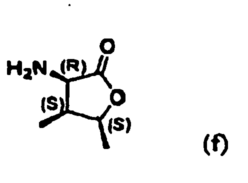 erhält, wenn man das N-geschützte Amin VIb verwendet;
• Lacton g 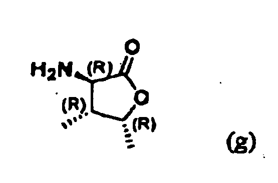 oder
• Lacton h 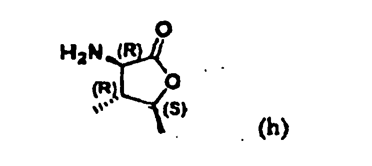 erhält, wenn man das N-geschützte Amin Vb verwendet;
- e) Hydrolysieren der Lactone, so dass man das gewünschte Stereo-isomer erhält:

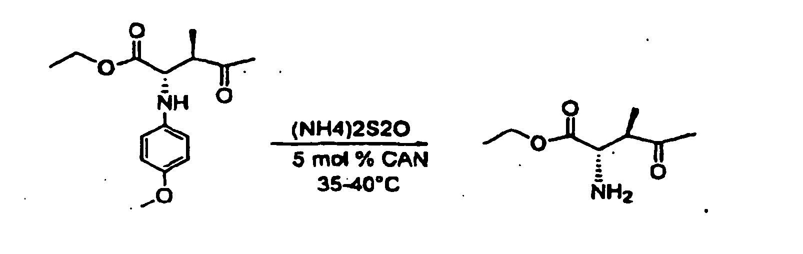

## Revendications

1. Procédé de préparation de diastéréoisomères de 4-hydroxyisoleucine de formule A :
CO₂H- CH(NH₂)- CH(CH₃)-CH(OH)-CH₃ (A)
comprenant :
- la déprotection d'une amine de formule B : 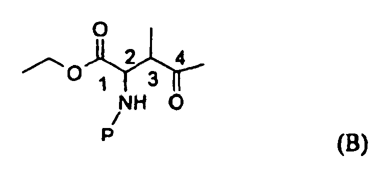
- la réduction et la lactonisation ensuite d'un dérivé d'amine intermédiaire de formule C : 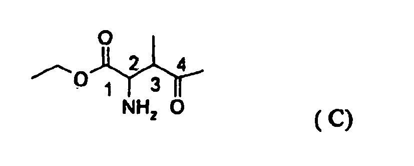 dont les substituants aux positions 2 et 3 sont soit R, S
ou S, S ; soit S, R ou R, R,
- l'hydrolyse de la lactone résultante de formule D : 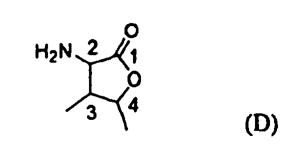 où les substituants aux positions 2, 3 et 4 sont
- S,R,S ou S,R,R, dans le cas de l'utilisation d'une amine protégée S,R
- S,S,S ou S,S,R, dans le cas de l'utilisation d'une amine protégée S,S
- R,S,R ou R,S,S, dans le cas de l'utilisation d'une amine protégée R,S
- R,R,R ou R,R,S, dans le cas de l'utilisation d'une amine protégée R,R
dans des conditions pour obtenir l'isomère souhaité de la 4-hydroxyisoleucine.

2. Procédé selon la revendication 1, dans lequel ladite lactone est obtenue dans un seul réacteur, par déprotection oxydante et réduction par un borohydrure.

3. Procédé selon la revendication 1, dans lequel ladite lactone est obtenue dans deux réacteurs par déprotection de l'amine avec un agent oxydant donnant lieu à un intermédiaire de formule C qui est isolé : 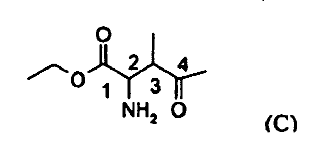 ledit dérivé intermédiaire C étant ensuite soumis à une réaction de réduction par un borohydrure pour obtenir les isomères souhaités.

4. Procédé selon l'une quelconque des revendications précédentes, comprenant l'étape consistant à utiliser ladite amine protégée de formule B, ayant des substituants R,S, respectivement aux positions 2 et 3.

5. Procédé selon l'une quelconque des revendications 1 à 3, comprenant l'étape consistant à utiliser ladite amine protégée de formule B, ayant des substituants S,S, respectivement aux positions 2 et 3.

6. Procédé selon l'une quelconque des revendications 1 à 3, comprenant l'étape consistant à utiliser ladite amine protégée de formule B, ayant des substituants R,R, respectivement aux positions 2 et 3.

7. Procédé selon l'une quelconque des revendications 1 à 3, comprenant l'étape consistant à utiliser ladite amine protégée de formule B, ayant des substituants S,R, respectivement aux positions 2 et 3.

8. Procédé selon l'une quelconque des revendications 1 à 7, dans lequel ladite amine protégée R,S ou S,R de formule B est obtenue par épimérisation de l'amine protégée correspondante S,S ou R,R.

9. Procédé selon la revendication 8, dans lequel ladite épimérisation est réalisée avec DBN/t-BuOMe.

10. Procédé selon l'une quelconque des revendications 1 à 9, dans lequel les amines protégées de formule B sont obtenues par condensation d'un dérivé de formule E : 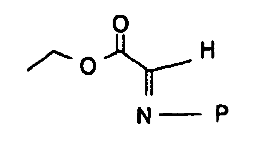 dans laquelle P représente un groupe de protection de groupe amino,
avec la 2-butanone 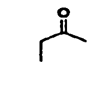 en présence de proline.

11. Procédé selon la revendication 10, dans lequel la proline est la L-proline.

12. Procédé selon la revendication 10, dans lequel la proline est la D-proline.

13. Procédé selon l'une quelconque des revendications précédentes, dans lequel le dérivé de formule E est obtenu par condensation d'un dérivé amino de formule F :
P-NH₂ (F)
avec l'éthylglyoxalate de formule G :

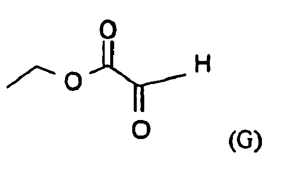

14. Procédé selon l'une quelconque des revendications précédentes pour préparer des diastéréoisomères de 4-hydroxyisoleucine qui comprend les étapes consistant à :
a) condenser un dérivé anisidine de formule I : 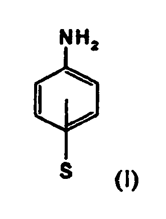 dans laquelle S représente un ou plusieurs substituants identiques ou différents choisis dans le groupe comprenant un groupe alkyle en C₁ à C₃, alcoxy avec un groupe alkyle en C₁ à C₃, ou aryloxy, halogéno tel que F, Cl, Br, I, un groupe nitro et des nitriles, avec l'éthylglyoxalate de formule II : 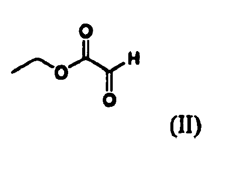 pour obtenir l'imine de formule III : 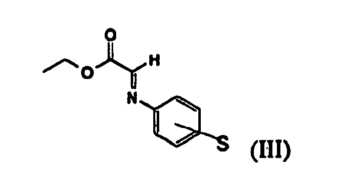
b) condenser l'imine de formule III avec la 2-butanone IV : 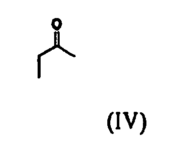 pour obtenir une amine protégée de formule Va ou Vb, 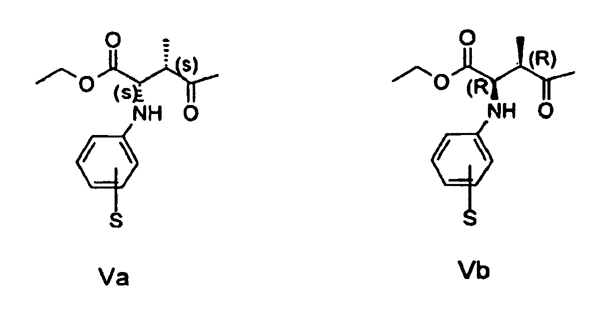 et si souhaité,
c) épimériser le dérivé de condensation résultant de formule Va ou Vb pour obtenir l'amine N-protégée de formule VIa ou VIb, respectivement : 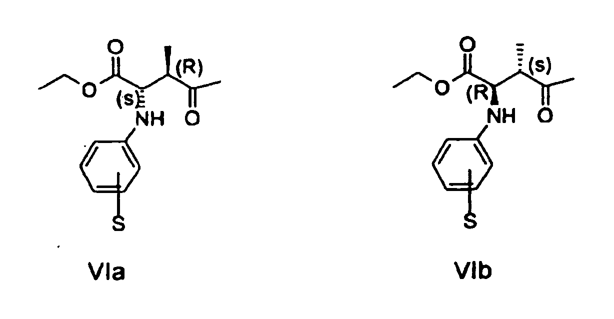
d) faire réagir ladite amine N-protégée Va ou Vb, ou VIa ou VIb, dans des conditions pour obtenir :
• la lactone a 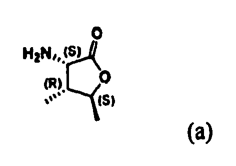
ou
• la lactone b 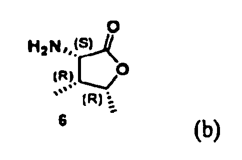 dans le cas de l'utilisation de l'amine N-protégée VIa
• la lactone c 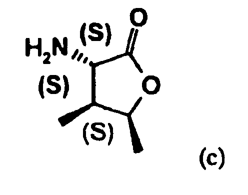
ou
• la lactone d 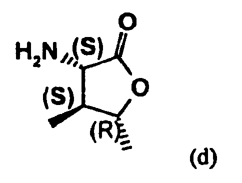 dans le cas de l'utilisation de l'amine N-protégée Va
ou
• la lactone e 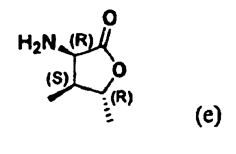
ou
• la lactone f 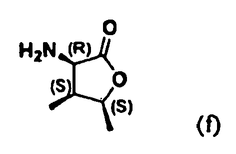 dans le cas de l'utilisation de l'amine N-protégée VIb
• la lactone g 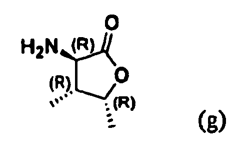
ou
• la lactone h 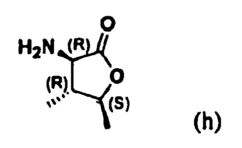 dans le cas de l'utilisation de l'amine N-protégée Vb
e) hydrolyser lesdites lactones pour obtenir le stéréoisomère souhaité :
